# EUROPEAN PATENT SPECIFICATION

(11) **EP 2 631 830 B1**
(45) Date of publication and mention of the grant of the patent: **08.09.2021**
(21) Application number: 13155358.8
(22) Date of filing: 15.02.2013
(51) Int. Cl.: G16H 20/30

(54) **Monitoring accumulated activity**
Überwachung von akkumulierter Aktivität
Surveillance de l'activité accumulée

(30) Priority: 17.02.2012 FI 20125185
(43) Date of publication of application: 28.08.2013
(73) Proprietor: Polar Electro Oy, 90440 Kempele (FI)
(72) Inventor: Erkkilä, Mika, 90440 Kempele (FI); Pääkkö, Tiina, 90440 Kempele (FI); Määttä, Harri, 90440 Kempele (FI)
(74) Representative: Kolster Oy Ab

(56) References cited:
- EP-A1- 1 849 504
- WO-A1-2004/016173
- US-A1- 2004 077 462
- US-A1- 2008 077 620
- US-A1- 2011 021 319
- None

## Description

### Field

The invention relates to the field of performance and activity monitors, such as sports computers and, particularly, to configuring such a device to monitor accumulated activity.

### Background

There are several types of exercise devices in the markets. Some of them are designed for athletes that concentrate heavily on their sports genres. Other devices are designed for monitoring general activity of a user to enable the user to ensure that he/she maintains a daily activity level. Such devices typically operate such that there is provided a target value and, then, the user's physical activity is measured with a measurement device attached to the user, and the measurement results are compared with the target value so as to determine whether or not the user has achieved the target.

US 2004/077462 discloses a method, system and program where at least one type of exercise indicator signal associated with exercise performed by a particular user is received from a particular exercise machine in a common transmittable data format at a portable computer system provided the particular user. Cumulative fitness activity is computed and stored for the particular user at the portable computer system utilizing the at least one type of exercise indicator signal and previously accumulated fitness activity data at the portable computer system, such that an independent portable computer system associated with the particular user monitors the real-time cumulative fitness activity of the particular user from at least one type of exercise indicator signal received from at least one exercise machine over a period of time.

US 2008/077620 discloses a system providing a lifestyle companion system. The lifestyle companion system can provide a platform to conduct a user interview. Based on the user interview responses, the system can suggest activities, references, and/or plug-in modules. During performance of activities, the system can provide audio and/or visual cues related to the activities and collect data indicative of the user's performance. Based on the collected data, the system can dynamically adapt the user's goals and/or activities the user is performing or will perform. In some embodiments of the present invention, the lifestyle companion system of the present invention can be applied to fitness, nutrition, and/or medical modules. The system also can be used to facilitate synchronous group activities.

### Brief description

The invention is defined by the independent claims. Embodiments are defined in the dependent claims.

According to an aspect, there is provided a computer program product embodied on a computer readable distribution medium arranged, when read by the computer, to cause execution of a computer process comprising: acquiring, in an activity monitoring apparatus, target accumulation parameters defining a target total amount of physical activity for a user to accumulate within an observation interval, wherein the amount of physical activity is defined in terms of an attribute measurable during the physical activity; distributing, in the activity monitoring apparatus, the target accumulation parameters into a plurality of subsets in a time domain such that each subset is associated with a time sub-interval and a target accumulation value, wherein the sum of the sub-intervals equals to the observation interval; and monitoring, in the activity monitoring apparatus, the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation.

According to yet another aspect, there is provided an apparatus comprising: at least one processor; and at least one memory including program instructions, wherein the at least one memory and the computer program code are configured, with the at least one processor, to cause the apparatus to: acquire target accumulation parameters defining a target total amount of physical activity for a user to accumulate within an observation interval, wherein the amount of physical activity is defined in terms of an attribute measurable during the physical activity; distribute the target accumulation parameters into a plurality of subsets in a time domain such that each subset is associated with a time sub-interval and a target accumulation value, wherein the sum of the sub-intervals equals to the observation interval; and monitor the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation.

In an example, the target accumulation values represent a cumulative distribution function of the total amount of physical activity, and the at least one memory and the computer program code are configured, with the at least one processor, to cause the apparatus to: acquire measured accumulation of the physical activity measured during said sub-interval and during all previous sub-intervals of the observation interval, if any; and compute the progress indicator by comparing the difference between the acquired measured accumulation of the physical activity and the target accumulation value of the current sub-interval.

In an example, the at least one memory and the computer program code are configured, with the at least one processor, to cause the apparatus to distribute the target accumulation parameters into the plurality of subsets unequally such that at least one of the sub-intervals is associated with higher target accumulation of physical activity than at least one other sub-interval. In an embodiment, the at least one memory and the computer program code are configured, with the at least one processor, to cause the apparatus to distribute the target accumulation parameters into a plurality of subsets unequally according to an initial estimate of a user activity accumulation distribution during the observation interval.

In the invention, the at least one memory and the computer program code are configured, with the at least one processor, to cause the apparatus to: determine a distribution of the measured accumulation of the physical activity within at least one previous observation interval preceding the observation interval by determining the measured accumulation of the physical activity during each sub-interval of the at least one previous observation interval; and distribute the target accumulation parameters into the plurality of subsets by using the determined distribution of the measured accumulation as a distribution model. In an embodiment, the at least one memory and the computer program code are configured, with the at least one processor, to cause the apparatus to offset the distribution of the target accumulation parameters of the observation interval such that the distribution of the target accumulation parameters of the observation interval is advanced with respect to the measured accumulation within the at least one previous observation interval by one sub-interval. In an embodiment, the at least one memory and the computer program code are configured, with the at least one processor, to cause the apparatus to average the measured accumulation of the physical activity over a plurality of said previous observation intervals.

In an example, the at least one memory and the computer program code are configured, with the at least one processor, to cause the apparatus to: acquire an initial distribution model for the target accumulation parameters; acquire a measured accumulation value of the physical activity for each sub-interval measured during the previous observation interval; determine a sub-interval in which a target accumulation value of the initial distribution model is the highest with respect to the measured accumulation value of a subsequent sub-interval and reducing the target accumulation value of the determined sub-interval in the initial distribution model; determine a sub-interval in which a target accumulation value of the initial distribution model is the lowest with respect to the measured accumulation value of a subsequent sub-interval and raising the target accumulation value of the determined sub-interval in the initial distribution model.

In an example, the at least one memory and the computer program code are configured, with the at least one processor, to cause the apparatus to acquire the measured the accumulated physical activity that comprises at least one of the following attributes of the user: heart rate, acceleration, speed, power, and energy consumption.

In an example, the at least one memory and the computer program code are configured, with the at least one processor, to cause the apparatus to: acquire a plurality of types of measured accumulation data for each sub-interval, wherein the plurality of types of measurement data are measured with different types of sensors; combine the plurality of types of measured accumulation data into a common format for each sub-interval; and monitor the physical activity of the user during each sub-interval by comparing the combined measured accumulation data with the corresponding target accumulation of the physical activity and by outputting a progress indicator indicating the measured accumulation of the physical activity with respect to the target accumulation physical activity.

### List of drawings

Embodiments of the present invention are described below, by way of example only, with reference to the accompanying drawings, in which
Figure 1 illustrates embodiments of an activity monitoring apparatus;
Figure 2 illustrates a flow diagram of a process for monitoring accumulation of physical activity of a user of the activity monitoring apparatus;
Figure 3 illustrates an example of a distribution of target accumulation parameters according to an embodiment of the invention;
Figure 4 illustrates a display illustrating a progress indicator according to an embodiment of the invention;
Figure 5 is a flow diagram of a process for determining the distribution of the target accumulation parameters according to history data according to an embodiment of the invention;
Figure 6 illustrates an example of a distribution of target accumulation parameters according to another embodiment of the invention;
Figure 7 is a flow diagram of a process for determining the distribution of the target accumulation parameters according to previously measured accumulation data according to an embodiment of the invention;
Figures 8A, 8B, and 8C illustrate modification of an initial distribution model according to an embodiment of the invention;
Figure 9 is a flow diagram of a process for measuring accumulation of physical activity from different types of measured accumulation data according to an embodiment of the invention;
Figure 10 is a block diagram of an apparatus according to an embodiment of the invention; and
Figure 11 illustrates an activity monitoring system according to an embodiment of the invention.

### Description of embodiments

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

Embodiments of the present invention relate to monitoring physical activity of a user over a time interval. The monitoring may be carried out by an activity monitoring apparatus associated with the user. An activity monitoring apparatus is an apparatus comprising means for monitoring user's physical activity.

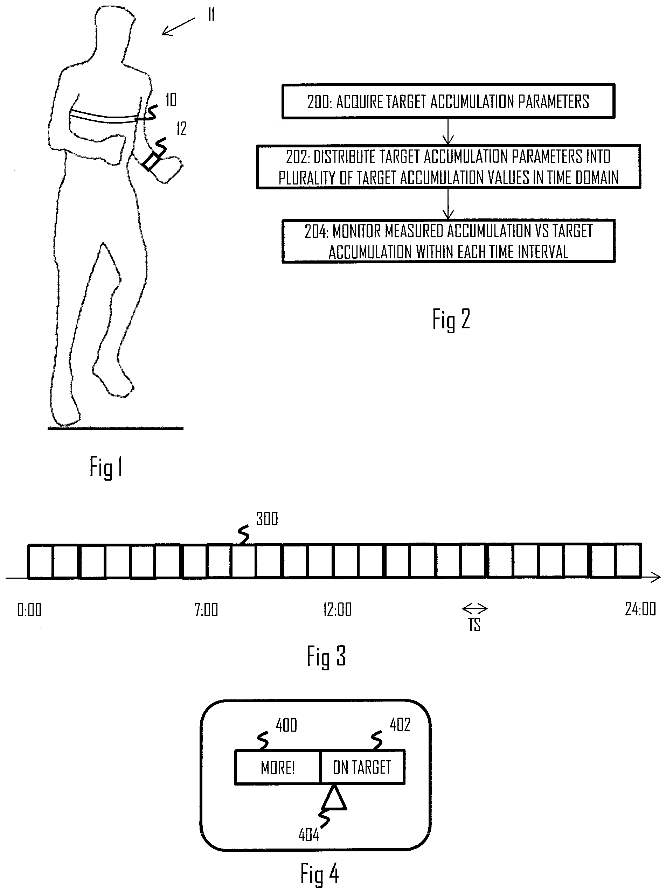

Referring to Figure 1, the activity monitoring apparatus may be a portable or wearable activity monitoring apparatus such as a wrist device 12 or a chest device 10 attached to the body of the user 11. The wrist device 12 may be a sports watch or a wrist computer similar to Polar FA20 activity monitor (registered trade mark) or Polar RS300 heart rate monitor (registered trade mark). The chest device 10 may be a heart rate transmitter similar to Polar Wearlink (registered trade mark). The activity monitoring apparatus may include several units, such as user interface unit and sensors which may be connected to each other wirelessly. The user interface unit comprises a user interface components, such as a display or audio interface.

In an embodiment, the user interface unit is a wrist device.

In an embodiment, the user interface unit a unit attachable to the user's head.

In an embodiment, the user interface unit comprises attachment means for attaching the user interface unit to a bicycle.

In an embodiment, the sensor is a heart activity sensor. The heart activity sensor may be based on an ECG detection from the user's skin or optical blood oximetry.

In an embodiment, the sensor is a motion sensor based on an accelerometer, a magnetometer or a gyroscope.

Let us now describe an embodiment of the invention for monitoring the physical activity of the user 11 with reference to a flow diagram of Figure 2. The process of Figure 2 may be carried out in the above-mentioned activity monitoring apparatus. Referring to Figure 2, target accumulation parameters defining a target total amount of physical activity for a user to accumulate within an observation interval are acquired in block 200. The amount of physical activity may be defined in terms of an attribute measurable during the physical activity. Examples of the attribute and how its accumulation may be determined are described below. In block 202, the target accumulation parameters are distributed into a plurality of subsets in a time domain such that each subset is associated with a time sub-interval and a target accumulation value, wherein the sum of the sub-intervals equals to the observation interval. The sum of the target accumulation sub-values may equal to the target total amount of physical activity, or the target accumulation sub-values may be distributed in an increasing order such that each target accumulation sub-value is a superposition of target accumulation sub-values of previous sub-intervals plus an additional target accumulation for the current sub-interval. The former describes density distribution of the target accumulation sub-values, while the latter cumulative distribution of the target accumulation sub-values, as will be described below. In block 204, the physical activity of the user 11 is monitored during a sub-interval by comparing a measured accumulation of the physical activity with the target accumulation derived from at least the target accumulation value of the sub-interval and by outputting a progress indicator indicating the measured accumulation of the physical activity with respect to the target accumulation.

In an embodiment, the target total amount of physical activity is defined in the form of the measurable attribute maintained with determined boundaries for a determined duration within the determined time interval.

In an embodiment, the measurable attribute is an activity measured by an acceleration sensor comprised in the activity monitoring apparatus or in communication with the activity monitoring apparatus. The measured acceleration may then be mapped to an activity metric such a metabolic equivalent of task (MET), energy consumption, etc. As a consequence, the measurable attribute per the time interval may be, for example, having MET above a threshold, e.g. 2.0 corresponding to walking intensity, for a determined duration within the determined time interval. Whenever the user's 11 activity is detected to increase above this threshold through the measurements, the user is considered to accumulate this type of activity for the duration the activity is above the threshold.

In an embodiment, the measurable attribute comprises user's energy expenditure. The energy expenditure may be measured from heart activity with an ECG-type (Electrocardiogram) measurement or with optical blood oximetry-based measurement with a sensor of the activity monitoring apparatus.

In an embodiment, the measurable attribute comprises accumulated time at given heart rate zone or combination of given heart rate zones. A heart rate zone is defined by a lower heart rate limit and a upper heart rate limit between which the measured heart rate is expected to locate in order to contribute to the accumulated time.

In an embodiment, the measurable attribute comprises an accumulated time at given speed zone. The speed may be running, walking or cycling speed. A speed zone is defined by a lower speed limit and an upper speed limit between which the measured speed is expected to locate in order to contribute to the accumulated time.

In an embodiment, the measurable attribute comprises an accumulated time at given power zone. The power is typically a measure characterizing the user's output power. The output power may be motion power or pedalling power, for example. A power zone is defined by a lower power limit and an upper power limit between which the measured power is expected to locate in order to contribute to the accumulated time. The motion power may be measured with an accelerometer fixed to the user. The cycling power may be measured with a cycling power sensor attached to the force transmission system of a bicycle.

In an embodiment, the measurable attribute comprises user's training load. The training load is a measure for the user's cardiovascular or mechanical load. Examples of training load characterizations are Trimp (Training Impulse), EPOC (Excess Post-exercise oxygen consumption), and Training Load by Polar Electro disclosed by US patent publication US 2011/021319.

The training load may be measured from heart activity with an ECG-type (Electrocardiogram) measurement or with optical blood oximetry-based measurement with a sensor of the activity monitoring apparatus.

In an embodiment, the total target accumulation parameters are distributed in the time domain to enable better evaluation of the progress of the activity within the determined time interval. The observation interval may be set sufficiently long to enable monitoring the user's everyday physical activity, for example. Therefore, in an embodiment the observation interval is one day. The observation interval may thus span beyond the duration of a single physical exercise that may last an hour or a couple of hours but it may be shorter than a training program comprising a plurality of exercises that span over several days or weeks. This type of evaluation enables the activity monitoring apparatus to compute the actual distribution of the user's activity within the determined time interval. The distribution of the target accumulation values over the observation interval may specify a default target for accumulating the activity, but it the user may in practice reach the total target accumulation with a different distribution. As a consequence, the activity monitoring apparatus may use the distribution of the target accumulation values only as a tool for evaluating the current progress with respect to the total target accumulation, and the conclusion of whether or not the total target accumulation was reached is made after the observation interval has elapsed.

The target accumulation parameters may be distributed uniformly in the time domain, as shown in the embodiment of Figure 3. In this embodiment, the observation interval is one day from 0:00 to 24:00, and this time interval is divided into one-hour sub-intervals TS. For each sub-interval, there is assigned an equal target accumulation value (denoted by a box in Figure 3, e.g. box 300) which may be acquired by dividing the total target accumulation by 24. The target accumulation value is illustrated by the height of a box. For example, if the target total activity is 2 hours, the height of the box in this case is 2/24=5 minutes. This means that the user shall me active at least 5 minutes during each hour in order to meet the target,

Let us now consider the operation of the activity monitoring apparatus when it is configured to monitor the activity of the user during the observation interval with reference to Figure 3 and 4. In an embodiment, the activity monitoring apparatus is configured to acquire measured accumulation of the physical activity measured during a current sub-interval and during all previous sub-intervals of the determined time interval, if any. Referring to Figure 3, let us assume that the current sub-interval is the sub-interval 300 from 8:00 to 9:00 and that the observation interval started at 0:00. As a consequence, this step may be triggered by the expiry of the current sub-interval 300 and it may comprise determining the accumulation of the physical activity up to the current sub-interval 300 including the current sub-interval 300. In another embodiment where the activity monitoring apparatus is configured to carry out the monitoring in real-time, the operation may be continuous such that the step comprises determining the accumulation of the physical activity up to the current time instant within the current sub-interval. By using the accumulated amount of the physical activity and the target total amount of the physical activity, it is possible to compute the ratio between the accumulated amount and the target total amount and, thus, to express the percentage of how much of the target total amount has already been accumulated.

The activity monitoring apparatus may also determine a progress index of the current sub-interval with respect to the total duration of the observation interval and, thus, it is possible to compute the ratio between the duration from the start of the observation interval and the total duration of the observation interval to express the percentage of how much of the observation interval has already lapsed.

By using these two ratios or values proportional to the ratios, the activity monitoring apparatus may compute the progress indicator. The progress indicator may be computed by evaluating the difference between the ratios, and the progress indicator may be displayed to the user in a user interface (see Figure 4) of the activity monitoring apparatus. If the ratio between the accumulated amount and the target total amount of physical activity is higher than the ratio between the duration from the start of the observation interval and the total duration of the determined time interval, it means that the user has performed according to the planned target distribution of the physical activity or even better. As a consequence, the activity monitoring apparatus may display the progress indicator 404 such that it indicates that the user is following the plan and is on target. The progress indicator 404 may be configured to indicate a display element 402 that shows to the user in words, image(s) or symbol(s) that the user is on target. On the other hand, if the ratio between the accumulated amount and the target total amount of physical activity is lower than the ratio between the duration from the start of the observation interval and the total duration of the determined time interval, it means that the user has not performed according to the planned target distribution of the physical activity and that the user has to improve his/her activity in order to reach the target. As a consequence, the activity monitoring apparatus may display the progress indicator 404 such that it indicates that the user is has to accumulate more activity. The progress indicator 404 may then be configured to indicate a display element 400 that shows to the user in words, image(s) or symbol(s) that the user is not on target but has to improve his/her activity accumulation. The progress indicator 404 may be a coarse indicator, e.g. a binary indicator indicating only two states: on the target and not on the target. In another embodiment, the progress indicator 404 may be a sliding indicator that slides between end points of a scale in multiple positions. The position of the indicator may be computed from the difference between the two above-mentioned ratios. When they are equal, the progress indicator 404 may be set in the halfway of the end points. When the user has accumulated the entire target total amount of physical activity, the progress indicator may be set to the end point indicating that the user has achieved the target 402. When the user has accumulated no physical activity, the progress indicator may be set to the end point indicating that the user is on not target 400. The positions between these points may be mapped according to the difference between the two ratios. The lower is the accumulated physical activity ratio with respect to the progress of the determined time interval, the closer the progress indicator 404 is to the end point of element 400. Similarly, the higher is the accumulated physical activity ratio with respect to the progress of the determined time interval, the closer the progress indicator 404 is to the end point of element 402.

Obviously, the above-described functionality may be encoded into the target accumulation values assigned to each sub-interval. Each sub-interval may be assigned with a cumulative value which represents the target amount of physical activity that should have been accumulated before the end of the sub-interval. As a consequence, the distribution of the target accumulation values represents a cumulative distribution function of the physical activity, wherein a target accumulation value assigned to a given sub-interval is equal to or higher than a target accumulation value of a previous sub-interval and equal to or lower than a target accumulation value of a subsequent sub-interval. For example, the target accumulation value of the last sub-interval equals to the target total accumulation value. This embodiment enables direct comparison between the measured accumulation and the target accumulation and reduces computation in the activity monitoring apparatus.

Figure 3 illustrates a simplified example of the distribution of the target accumulation parameters by using the uniform distribution. However, as the user's physical activity is not uniform over the observation interval that spans over several hours, the uniform distribution may not always provide an accurate estimate of whether or not the user is on target with respect to the total target of accumulated physical activity. For example, during the night the user's activity may be very low because of the sleep and, thus, the user may not accumulate any activity. On the other hand, when the user carries out an intensive exercise, the user may accumulate a high amount of physical activity during a relatively short time interval. As a consequence, an embodiment configures the activity monitoring apparatus to distribute the target accumulation parameters into the plurality of subsets non-uniformly such that at least one of the sub-intervals is associated with a higher target accumulation value than at least one other sub-interval. In an embodiment, the distribution is determined on the basis of user input through the user interface. The user input may comprise an input specifying the sub-intervals when the user sleeps and/or any other inactivity period. The activity monitoring apparatus may then assign no target accumulation value for these sub-intervals, which means that the user is not expected to perform any activity during these sub-intervals. The user input may comprise an input specifying the sub-intervals when the user is active, e.g. exercise periods. The activity monitoring apparatus may then assign at least some of the target accumulation values to these sub-intervals, which means that the user is expected to accumulate a higher activity during these sub-intervals.

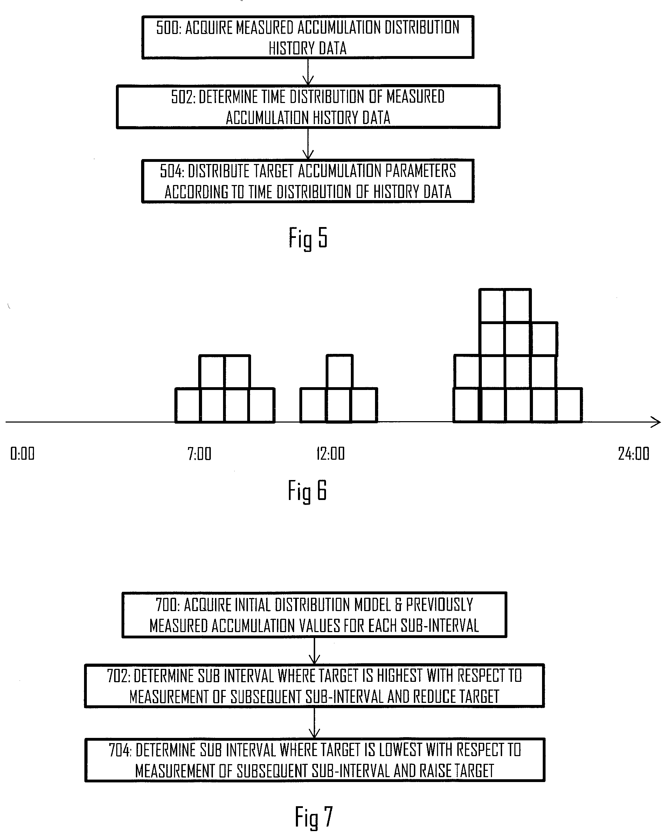

In another embodiment, the distribution is determined according to previously measured history data. The history data may be measured during one or more previous observation intervals. Figures 5 and 6 illustrate operation of such an embodiment. Referring to Figure 5, the activity monitoring apparatus may determine in block 500 a distribution of the measured accumulation of the physical activity within at least one previous observation interval preceding the current observation interval. The distribution may be acquired by determining the measured accumulation of the physical activity during each sub-interval of the at least one previous observation interval. From the measured accumulation of the physical activity during the sub-intervals of the previous observation intervals, the time distribution of the measured accumulation is determined in block 502. This history data shows the distribution of real accumulation of the physical activity within the observation interval. Block 502 may comprise averaging the measured accumulation values over a plurality of observation intervals in order to improve the accuracy. The averaging may comprise computing an average between the measured accumulation values of the same sub-interval of different previous observation intervals.

This distribution may serve as the distribution model used for distributing the target accumulation parameters into the plurality of subsets in block 504. Block 504 may comprise assigning the target accumulation values to the sub-intervals such that their distribution follows this distribution model determined in block 502. If the target total amount of the accumulated physical activity differs between the previous observation intervals and the present observation interval, the target accumulation values assigned to the sub-intervals may be scaled such that the distribution model remains essentially similar.

When the procedure is carried out for the first time and there is no measured history data available, the uniform distribution model may be used as an initial distribution model and/or user input may be used to modify the initial distribution model.

Referring to Figure 6 of an example of the distribution model determined in block 502, if the measured physical activity of the user shows that the user has accumulated no activity during the night, no target accumulation is assigned to these sub-intervals in block 504. Instead, a higher target accumulation value may be assigned to active periods, e.g. an active period in the morning starting from 6:00 until 10:00, another active period around the noon, and a high activity period in the early evening. The actual target accumulation values for each sub-interval may be derived from the distribution model.

In an embodiment, the process of Figure 5 comprises as a subroutine in block 502 offsetting the distribution model of the target accumulation parameters by one interval with respect to the measured distribution. In particular, the distribution model may be advanced with respect to the measured distribution model by one sub-interval. The reason is the inherent order of setting the target before achieving the target. As a consequence, the accuracy of the progress estimation is improved.

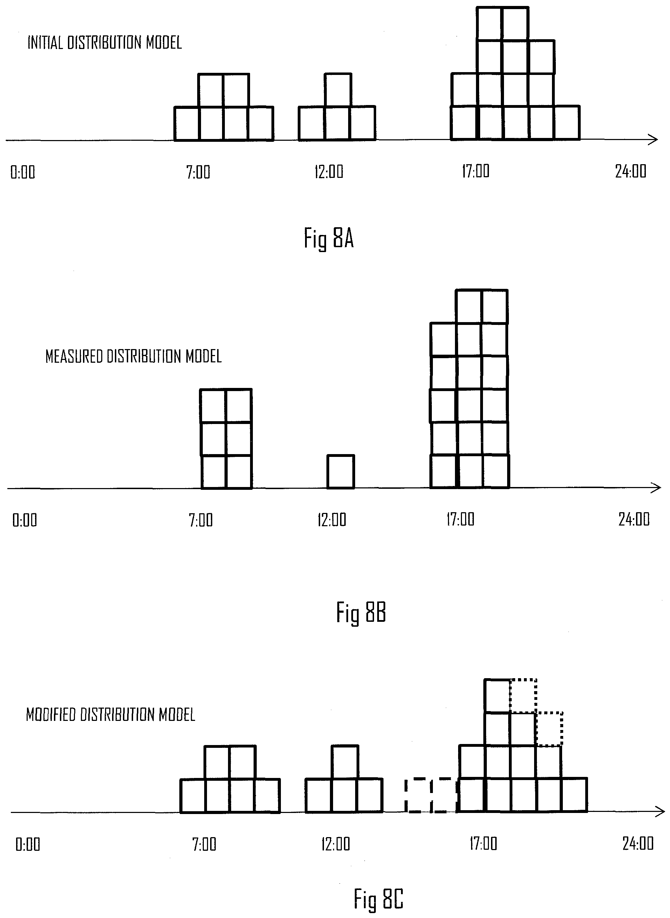

Another embodiment for using the history data to modify the distribution model is now described with reference to Figures 7 and 8. In this embodiment, the distribution model evolves between observation intervals such that the distribution model is modified towards the previously measured distribution model. Referring to Figure 7, an initial distribution model for the target accumulation parameters is acquired in block 700. The initial distribution model may be the distribution model used in the previous observation interval, or it may be derived in another manner, e.g. based on the user input. In block 700, the measured distribution model measured in connection with the previous observation interval is also acquired. The measured distribution model may comprise a measured accumulation value of the physical activity for each sub-interval measured during the previous observation interval.

In block 702, a sub-interval in which a target accumulation value of the initial distribution model is the highest with respect to the measured accumulation value of a subsequent sub-interval is determined. Then, the target accumulation value of the determined sub-interval is reduced in the initial distribution model. In block 704, a sub-interval in which a target accumulation value of the initial distribution model is the lowest with respect to the measured accumulation value of a subsequent sub-interval is determined. Then, the target accumulation value of the determined sub-interval is raised in the initial distribution model. As a result, a modified distribution model is achieved, wherein the modifications result in a distribution model that better matches with the measurements than the initial distribution model. This modified distribution model may then be used in block 202. The procedure of Figure 7 may thus be seen as an embodiment of block 202. Note that the embodiment of Figure 7 also implements the feature where the target accumulation values are advanced by one sub-interval with respect to the measured accumulation values. This feature is realized in the comparison in blocks 702 and 704 where the comparison is carried out between a target accumulation value of sub-interval X and a measured accumulation value of sub-interval X+1.

Figures 8A, 8B, and 8C illustrate the operation of the embodiment of Figure 7. Figure 8A illustrates the initial distribution model, and Figure 8 B illustrates a previously measured distribution model.

Let us assume that each box corresponds to 5 minutes of activity. In Figure 8A, the initial distribution includes three sub-intervals: one between 6 and 8 AM with 6x5=30 minutes of activity; one between 11 AM and 1 PM with 4x5=20 minutes of activity; and one between 4 PM and 9 PM with 14x5=1 hour 10 minutes of activity. Each of three sub-period includes variety in internal activity distribution.

In block 702 in which the activity monitoring apparatus determines the sub-interval where the target accumulation value is the highest with respect to the measured accumulation value of the subsequent sub-interval, the activity monitoring apparatus detects that the sub-interval is between 18:00 and 19:00 of the initial distribution model (four block of difference). On the other hand, in block 704 the activity monitoring apparatus detects that the sub-interval where the target accumulation value is the lowest with respect to the measured accumulation value of the subsequent sub-interval is the sub-interval between 15:00 and 16:00 of the initial distribution model. As a consequence, the target accumulation value is reduced in the sub-interval 18:00 to 19:00 (shown by dot-lined block in the sub-interval), and the target accumulation value is increased in the sub-interval 15:00 to 16:00 (shown by dash-lined block in the sub-interval). The value defining how much the target accumulation value is increased/decreased may be determined according to the design of the activity monitoring apparatus. In an embodiment, the value is proportional to the number of sub-intervals, and the value may be derived by dividing the target total accumulation value by the number of sub-intervals. For example, if the number of sub-intervals is 24 as in the illustrated examples, the value may be derived by dividing the target total accumulation value by 24.

Depending on how much the activity monitoring apparatus is configured to modify the initial distribution model, the activity monitoring apparatus may reiterate blocks 702 and 704 for a determined number of times by using the modified distribution model as the initial distribution model in a subsequent iteration. Figure 8C shows the result after two iterations, wherein a block representing the target accumulation is transferred from sub-interval 19:00 to 20:00 to sub-interval 14:00 to 15:00. The number of iterations may be determined to be sufficiently high to enable fast adaptation but sufficiently low that a single observation interval having significantly different distribution does not cause the activity monitoring apparatus to provide suboptimal progress estimation during a subsequent observation interval. In an embodiment, the number of iterations is two, as shown in Figure 8C.

Above, there has been described a number of examples of how to accumulate activity, and the embodiments described above have been described in the context where there activity monitoring apparatus defines a single target total accumulation value and a single target accumulation value for each sub-interval.

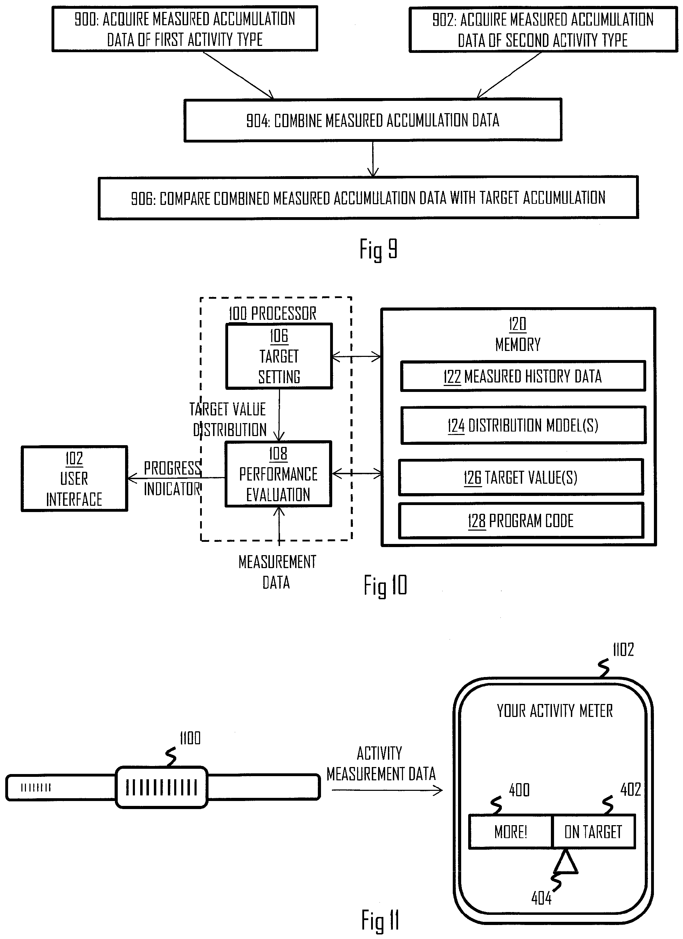

In an embodiment, the measured accumulation that is compared with the target accumulation is acquired from single type of measurements, e.g. heart rate measurements. In another embodiment, the measured accumulation is acquired through a plurality of types of measurements, e.g. heart rate measurements and acceleration measurements. The different types of measurements may be associated with different types of activities. Figure 9 illustrates a process for such an embodiment. Referring to Figure 9, the measured accumulation data of a first activity type is acquired in block 900, while the measured accumulation data of a second activity type is acquired in block 900. The measurement data of block 900 may be heart rate data acquired from a heart rate monitor attached to the user's body, while the measurement data of block 900 may be heart rate data acquired from an acceleration sensor attached to the user's body. Because the measured accumulation data of blocks 900 and 902 is of different types, the activity monitoring apparatus may be configured to combine the measured accumulation data in order to make them comparable. The combining is carried out in block 904. The combining may comprise converting at least one of the two types of measured accumulation data into a format which is common to both types of measured accumulation data. The format may be energy consumption, for example. All types of measured activity accumulation data including the above-mentioned examples of accumulation data may be converted into a format of energy consumption according to state-of-the-art conversion techniques. The target total accumulation value may also be in the same format to enable the comparison between the combined measured accumulation data and the target total accumulation data. The comparison is made in block 906, and the comparison may comprise at least some of the steps described above in connection with block 204.

In another embodiment, a unique target total accumulation value is assigned to each of the plurality of types of measurement data, and the target accumulation parameters comprise at least a first and a second target total accumulation value. The first and second target total accumulation value may each be associated with a different type of activity. Then, the first target total accumulation value and the second target total accumulation value may be divided into the plurality of subsets in a time domain separately such that each subset is associated with a time sub-interval, a first target accumulation value, and a second target accumulation value. Then, the physical activity of the user may be monitored during each sub-interval by comparing measured accumulation of each type of physical activity with the corresponding target accumulation value, and by deriving a separate progress indicator value for each type of activity. These progress indicators may then be combined to obtain the comparison results, and by outputting a total progress indicator indicating the measured total accumulation of the physical activity with respect to the target accumulation parameters. It should be appreciated that there are other, equivalent procedures for taking a plurality of activity types into account according to the principles of these embodiments.

Figure 10 illustrates a block diagram of an apparatus according to an embodiment of the invention. The apparatus may be the activity monitoring apparatus or it may be comprised in the activity monitoring apparatus. As mentioned above, the activity monitoring apparatus may be a portable or wearable device attachable to the user's body. The activity monitoring apparatus may comprise at least one sensor configured to measure the accumulation data, as described above, or the activity monitoring apparatus may be configured to establish a communication link with said at least one sensor when the at least one sensor is disposed in a different apparatus, e.g. in a different casing.

Referring to Figure 10, the apparatus comprises at least one processor 100 and at least one memory 120 including a computer program code 128, wherein the at least one memory 120 and the computer program code 128 are configured, with the at least one processor 100, to cause the apparatus to carry out any one of the above-described embodiments. The memory 120 may further store any measured history data 122 comprising accumulation data measured during previous observation interval(s), at least one distribution model 124 for distributing the target accumulation parameters, e.g. a previously used distribution model or an initial distribution model, and the target accumulation parameters 126.

The processor 100 may comprise a target setting circuitry 106 configured to retrieve from the memory the distribution model 124 and the target accumulation parameters 126 and to distribute the target accumulation values into the sub-intervals according to the distribution determined in the above-described manner. The target setting circuitry 106 may then indicate the completion of the preparations for monitoring the physical activity of the user, and it may output the target value distribution to a performance evaluation circuitry 108. The performance evaluation circuitry 108 may acquire measurement data from the sensor(s) or from the memory 120 and convert the measurement data into the measured accumulation data. Let us note that the data acquired from the sensor(s) may be raw data representing the heart rate, acceleration, speed, power, energy consumption, etc. and the raw data may need to be processed into the measured accumulation data. The processing may be carried out by determining the boundaries for the accumulation data, e.g. the heart rate limits or acceleration limits that cause the accumulation of the measurement data. The performance evaluation circuitry 108 may comprise or be in communication with a timer in order to determine the duration in which the measurement data was in the predefined limits so as to determine the measured accumulation duration. The performance evaluation circuitry 108 may further carry out the comparison between the measured accumulation data and the target accumulation associated with the current sub-interval and derive the progress indicator. The performance evaluation circuitry 108 may then output the progress indicator to the user interface 102 configured to map the received progress indicator into an appropriate display element. The performance evaluation circuitry 108 may further store the measured accumulation data in the memory 120 as new history data 122.

In summary, the apparatus may comprise at least one processor 100; and at least one memory 120 including program instructions, wherein the at least one memory 120 and the computer program code are configured, with the at least one processor 100, to cause the apparatus to carry out any one of the embodiments of the activity monitor apparatus.

As used in this application, the term 'circuitry' refers to all of the following: (a) hardware-only circuit implementations such as implementations in only analog and/or digital circuitry; (b) combinations of circuits and software and/or firmware, such as (as applicable): (i) a combination of processor(s) or processor cores; or (ii) portions of processor(s)/software including digital signal processor(s), software, and at least one memory that work together to cause an apparatus to perform specific functions; and (c) circuits, such as a microprocessor(s) or a portion of a microprocessor(s), that require software or firmware for operation, even if the software or firmware is not physically present.

This definition of 'circuitry' applies to all uses of this term in this application. As a further example, as used in this application, the term "circuitry" would also cover an implementation of merely a processor (or multiple processors) or portion of a processor, e.g. one core of a multi-core processor, and its (or their) accompanying software and/or firmware. The term "circuitry" would also cover, for example and if applicable to the particular element, a baseband integrated circuit, an application-specific integrated circuit (ASIC), and/or a field-programmable grid array (FPGA) circuit for the apparatus according to an embodiment of the invention.

The processes or methods described in Figures 4 to 8 may also be carried out in the form of a computer process defined by a computer program. The computer program may be in source code form, object code form, or in some intermediate form, and it may be stored in some sort of carrier, which may be any entity or device capable of carrying the program. Such carriers include transitory and/or non-transitory computer media, e.g. a record medium, computer memory, read-only memory, electrical carrier signal, telecommunications signal, and software distribution package. Depending on the processing power needed, the computer program may be executed in a single electronic digital processing unit or it may be distributed amongst a number of processing units.

Figure 11 illustrates yet another embodiment of an activity monitoring system. Referring to Figure 11, the system comprises an activity measurement device 1100 and a user interface device 1102. One or both of the devices 1100, 1102 may operate as the activity monitoring apparatus described above, and they may be provided in separate casings with no fixed mechanical contact between the devices 1100, 1102. The activity measurement device 1100 may measure the user's activity with a motion sensor comprised in the device 1100, and it may send the measured activity measurement data to the user interface device 1102. The activity measurement data may be raw data from which the user interface device 1102 may compute the activity and the accumulation of the physical activity. The activity measurement device 1100 may be a strap attachable to the user's wrist, for example, and comprising at least one motion sensor and a communication circuitry to communicate with the user interface device 1102. The activity measurement device 110 may comprise a display. The display may be a simplified display consisting of a series of led (light emitting diode) indicators. As a consequence, the activity measurement device 1100 may be without a complex display element such as a liquid crystal display. In an embodiment where the activity measurement device 1100 operates as the activity monitoring device, the activity measurement device may switch led indicators on or off in proportion to how well the accumulated physical activity compares with the target accumulation of the physical activity. For example, at least one led indicator having a first colour may be lit when measured accumulation of the physical activity matches with or exceeds the target accumulation. On the contrary, at least one led indicator having a second colour, different from the first colour, may be lit when measured accumulation of the physical activity is below the target accumulation, and/or at least one led indicator of the first colour may be shut down.

As described above, the activity measurement device 1100 may transmit the activity measurement data to the user interface device 1102. The transmission may be carried out in a continuous manner during the physical activity and during an observation interval, e.g. in real time, or the activity measurement data measured over a longer interval, e.g. over one or more observation intervals or sub-intervals, may be transmitted in a bundle. In the previous case, the user interface device 1102 may operate as the activity monitoring device during the observation intervals and monitor the accumulated activity with respect to the target accumulation in real time during the observation intervals. The user interface device may display the progress indicator illustrated in Figure 4, for example. In the embodiment where the measurement device 1100 provides the activity measurement data after the observation interval, the monitoring of the activity accumulated during the observation interval may be carried out after the observation interval. Similar progress indicator may still be used to indicate the accumulated activity versus the target accumulation.

The user interface device 1102 may be a computer or a portable computer such as a personal computer (PC), a laptop computer, a personal digital assistant (PDA), a palm computer, a mobile phone or a smart phone, or a tablet computer, for example. The user interface device 1102 may comprise a more complex display than that of the activity measurement device 1100. The more complex display may comprise a liquid crystal display (LCD), e.g. an active matrix display, a passive matrix display, or an AMOLED (active matrix organic LED) display. The user interface device 1102 may receive the raw activity measurement data from the activity measurement device 1100 and compute various statistics from the received raw data. The user interface device 1102 may compute, for example, measured activity accumulation distribution with respect to the target accumulation and display it to the user according to the embodiments described above. The user interface device 1102 may compute other statistics, e.g. activity history data spanning further into the past than the observation interval of the activity accumulation. The activity history data may represent long-term activity accumulation and/or distribution in time.

In an embodiment, the activity measurement device 1100 measures the activity measurement data, transmits it to the user interface device 1102, the user interface device 1102 computes the activity accumulation and compares it with the target accumulation in the above-described manner, and transmits a control signal to the activity measurement device 1100 on the basis of the comparison. The contents of the control signal may be determined according to how the measured activity accumulation measures with the target accumulation, and the control signal may control the user interface of the activity measurement device 1100 to illustrate the result of the comparison. The control signal instructs the activity measurement device 1100 to light or shut down at least some of the led indicators of the activity measurement device according to the result of the comparison. As a consequence, the user interface of the activity measurement device 1100 may serve as a simplified display to display the results of the comparison, e.g. through the led indicators, and the display of the user interface device 1102 may be used to illustrate the measured activity in a more complex manner through the LCD display, e.g. with the progress indicator of Figure 4.

An aspect provides a method comprising: acquiring, in an activity monitoring apparatus, target accumulation parameters defining a target total amount of physical activity for a user to accumulate within an observation interval, wherein the amount of physical activity is defined in terms of an attribute measurable during the physical activity; distributing, in the activity monitoring apparatus, the target accumulation parameters into a plurality of subsets in a time domain such that each subset is associated with a time sub-interval and a target accumulation value, wherein the sum of the sub-intervals equals to the observation interval; and monitoring, in the activity monitoring apparatus, the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation.

In an embodiment, the target accumulation values represent a cumulative distribution function of the total amount of physical activity, and the monitoring comprises: acquiring measured accumulation of the physical activity measured during said sub-interval and during all previous sub-intervals of the observation interval, if any; and computing the progress indicator by comparing the difference between the acquired measured accumulation of the physical activity and the target accumulation value of the current sub-interval.

In an embodiment, the method further comprises distributing the target accumulation parameters into the plurality of subsets unequally such that at least one of the sub-intervals is associated with higher target accumulation of physical activity than at least one other sub-interval. The target accumulation parameters may be distributed into a plurality of subsets unequally according to an initial estimate of a user activity accumulation distribution during the observation interval.

In an embodiment, the method further comprises: determining a distribution of the measured accumulation of the physical activity within at least one previous observation interval preceding the observation interval by determining the measured accumulation of the physical activity during each sub-interval of the at least one previous observation interval; distributing the target accumulation parameters into the plurality of subsets by using the determined distribution of the measured accumulation as a distribution model. The distribution of the target accumulation parameters of the observation interval may be offset such that the distribution of the target accumulation parameters of the observation interval is advanced with respect to the measured accumulation within the at least one previous observation interval by one sub-interval. The measured accumulation of the physical activity may be averaged over a plurality of said previous observation intervals.

In an embodiment, the distribution comprises: acquiring an initial distribution model for the target accumulation parameters; acquiring a measured accumulation value of the physical activity for each sub-interval measured during the previous observation interval; determining a sub-interval in which a target accumulation value of the initial distribution model is the highest with respect to the measured accumulation value of a subsequent sub-interval and reducing the target accumulation value of the determined sub-interval in the initial distribution model; determining a sub-interval in which a target accumulation value of the initial distribution model is the lowest with respect to the measured accumulation value of a subsequent sub-interval and raising the target accumulation value of the determined sub-interval in the initial distribution model.

In an embodiment, the accumulated physical activity is measured by measuring at least one of the following attributes of the user: heart rate, acceleration, speed, power, and energy consumption.

In an embodiment, the method further comprises: acquiring a plurality of types of measured accumulation data for each sub-interval, wherein the plurality of types of measurement data are measured with different types of sensors; combining the plurality of types of measured accumulation data into a common format for each sub-interval; and monitoring the physical activity of the user during each sub-interval by comparing the combined measured accumulation data with the corresponding target accumulation of the physical activity and by outputting a progress indicator indicating the measured accumulation of the physical activity with respect to the target accumulation physical activity.

It will be obvious to a person skilled in the art that, as technology advances, the inventive concept can be implemented in various ways. The invention and its embodiments are not limited to the examples described above but may vary within the scope of the claims.

## Claims

1. An apparatus (1100, 1102) comprising:
at least one processor (100); and
at least one memory (120) including computer program code (128), wherein the at least one memory and the computer program code are configured, with the at least one processor, to cause the apparatus to:
acquire (200) target accumulation parameters defining a target total amount of physical activity for a user to accumulate within an observation interval, wherein the amount of physical activity is defined in terms of an attribute measurable during the physical activity;
perform a time distribution (202) of the target accumulation parameters into a plurality of subsets in a time domain such that each subset is associated with a time sub-interval and a target accumulation value, wherein the sum of the sub-intervals equals to the observation interval; and
monitor (204) the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation,
**characterized in that** the at least one memory and the computer program code are configured, with the at least one processor, to cause the apparatus further to:
determine (700) a time distribution of the measured accumulation of the physical activity over at least one previous observation interval preceding the observation interval by determining the measured accumulation of the physical activity during each sub-interval of the at least one previous observation interval; and
modify (702, 704) the time distribution of the target accumulation parameters in the plurality of subsets towards the determined time distribution of the measured accumulation of the physical activity during the at least one previous observation interval.

2. The apparatus of claim 1, wherein the target accumulation values represent a cumulative distribution function of the total amount of physical activity, and the at least one memory and the computer program code are configured, with the at least one processor, to cause the apparatus to:
acquire measured accumulation of the physical activity measured during said sub-interval and during all previous sub-intervals of the observation interval, if any; and
compute the progress indicator by comparing the difference between the acquired measured accumulation of the physical activity and the target accumulation value of the current sub-interval.

3. The apparatus of claim 1 or 2, wherein the at least one memory and the computer program code are configured, with the at least one processor, to cause the apparatus to perform the time distribution (202) of the target accumulation parameters into the plurality of subsets unequally such that at least one of the sub-intervals is associated with higher target accumulation of physical activity than at least one other sub-interval.

4. The apparatus of claim 3, wherein the at least one memory and the computer program code are configured, with the at least one processor, to cause the apparatus to perform the time distribution (202) of the target accumulation parameters into the plurality of subsets unequally according to an initial estimate of a user activity accumulation distribution during the observation interval.

5. The apparatus of claim 1, wherein the at least one memory and the computer program code are configured, with the at least one processor, to cause the apparatus to offset the time distribution of the target accumulation parameters of the observation interval such that the distribution of the target accumulation parameters of the observation interval is advanced with respect to the measured accumulation within the at least one previous observation interval by one sub-interval.

6. The apparatus of claim 1 or 5, wherein the at least one memory and the computer program code are configured, with the at least one processor, to cause the apparatus to average the measured accumulation of the physical activity over a plurality of said previous observation intervals.

7. The apparatus of any preceding claim, wherein the at least one memory and the computer program code are configured, with the at least one processor, to cause the apparatus to:
acquire an initial distribution model for the target accumulation parameters;
acquire a measured accumulation value of the physical activity for each sub-interval measured during the previous observation interval;
determine a sub-interval in which a target accumulation value of the initial distribution model is the highest with respect to the measured accumulation value of a subsequent sub-interval and reducing the target accumulation value of the determined sub-interval in the initial distribution model;
determine a sub-interval in which a target accumulation value of the initial distribution model is the lowest with respect to the measured accumulation value of a subsequent sub-interval and raising the target accumulation value of the determined sub-interval in the initial distribution model.

8. The apparatus of any preceding claim, wherein the at least one memory and the computer program code are configured, with the at least one processor, to cause the apparatus to acquire the measured accumulated physical activity that comprises at least one of the following attributes of the user: heart rate, acceleration, speed, power, and energy consumption.

9. The apparatus of any preceding claim, wherein the at least one memory and the computer program code are configured, with the at least one processor, to cause the apparatus to:
acquire a plurality of types of measured accumulation data for each sub-interval, wherein the plurality of types of measurement data are measured with different types of sensors;
combine the plurality of types of measured accumulation data into a common format for each sub-interval; and
monitor the physical activity of the user during each sub-interval by comparing the combined measured accumulation data with the corresponding target accumulation of the physical activity and by outputting a progress indicator indicating the measured accumulation of the physical activity with respect to the target accumulation physical activity.

10. The apparatus of any preceding claim, wherein the apparatus comprises a motion sensor arranged to measure the physical activity.

11. The apparatus of claim 10, wherein the at least one memory and the computer program code are configured, with the at least one processor, to cause the apparatus to communicate at least the measured accumulation of the physical activity to another apparatus.

12. The apparatus of any preceding claim 1 to 9, wherein the at least one memory and the computer program code are configured, with the at least one processor, to cause the apparatus to receive activity measurement data from another apparatus during the monitored sub-interval.

13. The apparatus of any preceding claim 1 to 9, wherein the at least one memory and the computer program code are configured, with the at least one processor, to cause the apparatus to receive activity measurement data from another apparatus after the monitored sub-interval and performing said monitoring upon receiving the activity measurement data.

14. A computer program product embodied on a computer-readable distribution medium arranged, when read by a computer, to cause execution of a computer process comprising:
acquiring (200) target accumulation parameters defining a target total amount of physical activity for a user to accumulate within an observation interval, wherein the amount of physical activity is defined in terms of an attribute measurable during the physical activity;
performing a time distribution (202) of the target accumulation parameters into a plurality of subsets in a time domain such that each subset is associated with a time sub-interval and a target accumulation value, wherein the sum of the sub-intervals equals to the observation interval; and
monitoring (204) the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation
**characterized by** the computer process further comprising:
determining (700) a time distribution of the measured accumulation of the physical activity over at least one previous observation interval preceding the observation interval by determining the measured accumulation of the physical activity during each sub-interval of the at least one previous observation interval; and
modifying (702, 704) the time distribution of the target accumulation parameters in the plurality of subsets towards the determined distribution of the measured accumulation of the physical activity during the at least one previous observation interval.

15. An apparatus (1100, 1102) comprising:
at least one processor (100); and
at least one memory (120) including computer program code (128), wherein the at least one memory and the computer program code are configured, with the at least one processor, to cause the apparatus to:
acquire (200) target accumulation parameters defining a target total amount of physical activity for a user to accumulate within an observation interval, wherein the amount of physical activity is defined in terms of an attribute measurable during the physical activity;
perform a time distribution (202) of the target accumulation parameters into a plurality of subsets in a time domain such that each subset is associated with a time sub-interval and a target accumulation value, wherein the sum of the sub-intervals equals to the observation interval; and
monitor (204) the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation,
**characterized in that** the at least one memory and the computer program code are configured, with the at least one processor, to cause the apparatus further to:
determine (500) a time distribution of the measured accumulation of the physical activity over at least one previous observation interval preceding the observation interval by determining the measured accumulation of the physical activity during each sub-interval of the at least one previous observation interval; and
performing the time distribution (504) of the target accumulation parameters into the plurality of subsets in the time domain such that the time distribution (504) of the target accumulation parameters follows the determined time distribution of the measured accumulation of the physical activity during the at least one previous observation interval.

16. A computer program product embodied on a computer-readable distribution medium arranged, when read by a computer, to cause execution of a computer process comprising:
acquiring (200) target accumulation parameters defining a target total amount of physical activity for a user to accumulate within an observation interval, wherein the amount of physical activity is defined in terms of an attribute measurable during the physical activity;
performing a time distribution (202) of the target accumulation parameters into a plurality of subsets in a time domain such that each subset is associated with a time sub-interval and a target accumulation value, wherein the sum of the sub-intervals equals to the observation interval; and
monitoring (204) the physical activity of the user during a sub-interval by comparing a measured accumulation of the physical activity with the target accumulation derived from at least the target accumulation value of the sub-interval and by outputting a progress indicator indicating the measured accumulation of the physical activity with respect to the target accumulation
**characterized by** the computer process further comprising:
determining (500) a time distribution of the measured accumulation of the physical activity over at least one previous observation interval preceding the observation interval by determining the measured accumulation of the physical activity during each sub-interval of the at least one previous observation interval; and
performing the time distribution (504) of the target accumulation parameters into the plurality of subsets in the time domain such that the time distribution (504) of the target accumulation parameters follows the determined time distribution of the measured accumulation of the physical activity during the at least one previous observation interval.

## Patentansprüche

1. Vorrichtung (1100, 1102), die Folgendes umfasst:
mindestens einen Prozessor (100) und
mindestens einen Speicher (120), der Computerprogrammcode (128) beinhaltet, wobei der mindestens eine Speicher und der Computerprogrammcode dazu ausgelegt sind, die Vorrichtung mit dem mindestens einen Prozessor zu Folgendem zu veranlassen:
Erfassen (200) von Zielakkumulationsparametern, die eine Zielgesamtmenge einer physischen Aktivität für einen Benutzer definiert, die innerhalb eines Beobachtungsintervalls zu akkumulieren ist, wobei die Menge der physischen Aktivität mit Bezug auf ein Attribut, das während der physischen Aktivität messbar ist, definiert ist;
Durchführen einer Zeitverteilung (202) der Zielakkumulationsparameter in eine Vielzahl von Untersätzen in einer Zeitdomäne, derart, dass jeder Untersatz mit einem Zeitunterintervall und einem Zielakkumulationswert verknüpft ist, wobei die Summe der Unterintervalle gleich dem Beobachtungsintervall ist; und
Überwachen (204) der physischen Aktivität des Benutzers während eines Unterintervalls durch Vergleichen einer gemessenen Akkumulation der physischen Aktivität mit der Zielakkumulation, die mindestens aus dem Zielakkumulationswert des Unterintervalls abgeleitet wird, und durch Ausgeben eines Fortschrittsindikators, der die gemessene Akkumulation der physischen Aktivität mit Bezug auf die Zielakkumulation anzeigt,
**dadurch gekennzeichnet, dass** der mindestens eine Speicher und der Computerprogrammcode dazu ausgelegt sind, die Vorrichtung mit dem mindestens einen Prozessor ferner zu Folgendem zu veranlassen:
Bestimmen (700) einer Zeitverteilung der gemessenen Akkumulation der physischen Aktivität über mindestens ein vorheriges Beobachtungsintervall, das dem Beobachtungsintervall vorausgeht, durch Bestimmen der gemessenen Akkumulation der physischen Aktivität während jedes Unterintervalls des mindestens einen vorherigen Beobachtungsintervalls; und
Modifizieren (702, 704) der Zeitverteilung der Zielakkumulationsparameter in der Vielzahl von Untersätzen zur bestimmten Zeitverteilung der gemessenen Akkumulation der physischen Aktivität während des mindestens einen vorherigen Beobachtungsintervalls.

2. Vorrichtung nach Anspruch 1, wobei die Zielakkumulationswerte eine kumulative Verteilungsfunktion der Gesamtmenge der physischen Aktivität repräsentieren und der mindestens eine Speicher und der Computerprogrammcode dazu ausgelegt sind, die Vorrichtung mit dem mindestens einen Prozessor zu Folgendem zu veranlassen:
Erfassen einer gemessenen Akkumulation der physischen Aktivität, die während des Unterintervalls und während aller vorherigen Unterintervalle des Beobachtungsintervalls, sofern zutreffend, gemessen wurde; und
Berechnen des Fortschrittsindikators durch Vergleichen der Differenz zwischen der erfassten gemessenen Akkumulation der physischen Aktivität und dem Zielakkumulationswert des aktuellen Unterintervalls.

3. Vorrichtung nach Anspruch 1 oder 2, wobei der mindestens eine Speicher und der Computerprogrammcode dazu ausgelegt sind, die Vorrichtung mit dem mindestens einen Prozessor zu veranlassen, die Zeitverteilung (202) der Zielakkumulationsparameter uneinheitlich in die Vielzahl von Untersätzen durchzuführen, derart, dass mindestens eines der Unterintervalle mit einer höheren Zielakkumulation der physischen Aktivität verknüpft ist als mindestens ein anderes Unterintervall.

4. Vorrichtung nach Anspruch 3, wobei der mindestens eine Speicher und der Computerprogrammcode dazu ausgelegt sind, die Vorrichtung mit dem mindestens einen Prozessor zu veranlassen, die Zeitverteilung (202) der Zielakkumulationsparameter gemäß einer anfänglichen Schätzung einer Benutzeraktivitätsakkumulationsverteilung während des Beobachtungsintervalls uneinheitlich in die Vielzahl von Untersätzen durchzuführen.

5. Vorrichtung nach Anspruch 1, wobei der mindestens eine Speicher und der Computerprogrammcode dazu ausgelegt sind, die Vorrichtung mit dem mindestens einen Prozessor zu veranlassen, die Zeitverteilung der Zielakkumulationsparameter des Beobachtungsintervalls derart zu versetzen, dass die Verteilung der Zielakkumulationsparameter des Beobachtungsintervalls mit Bezug auf die gemessene Akkumulation in dem mindestens einen vorherigen Beobachtungsintervall um ein Unterintervall vorgerückt wird.

6. Vorrichtung nach Anspruch 1 oder 5, wobei der mindestens eine Speicher und der Computerprogrammcode dazu ausgelegt sind, die Vorrichtung mit dem mindestens einen Prozessor zu veranlassen, die gemessene Akkumulation der physischen Aktivität über eine Vielzahl der vorherigen Beobachtungsintervalle zu mitteln.

7. Vorrichtung nach einem der vorhergehenden Ansprüche, wobei der mindestens eine Speicher und der Computerprogrammcode dazu ausgelegt sind, die Vorrichtung mit dem mindestens einen Prozessor zu Folgendem zu veranlassen:
Erfassen eines anfänglichen Verteilungsmodells für die Zielakkumulationsparameter;
Erfassen eines gemessenen Akkumulationswertes der physischen Aktivität für jedes Unterintervall, der während des vorherigen Beobachtungsintervalls gemessen wurde;
Bestimmen eines Unterintervalls, in dem ein Zielakkumulationswert des anfänglichen Verteilungsmodells mit Bezug auf den gemessenen Akkumulationswert eines nachfolgenden Unterintervalls der höchste ist, und Reduzieren des Zielakkumulationswertes des bestimmten Unterintervalls im anfänglichen Verteilungsmodell;
Bestimmen eines Unterintervalls, in dem ein Zielakkumulationswert des anfänglichen Verteilungsmodells mit Bezug auf den gemessenen Akkumulationswert eines nachfolgenden Unterintervalls der niedrigste ist, und Erhöhen des Zielakkumulationswertes des bestimmten Unterintervalls im anfänglichen Verteilungsmodell.

8. Vorrichtung nach einem der vorhergehenden Ansprüche, wobei der mindestens eine Speicher und der Computerprogrammcode dazu ausgelegt sind, die Vorrichtung mit dem mindestens einen Prozessor zu veranlassen, die gemessene akkumulierte physische Aktivität, die mindestens eines der folgenden Attribute des Benutzers umfasst, zu erfassen: Herzfrequenz, Beschleunigung, Geschwindigkeit, Leistung und Energieverbrauch.

9. Vorrichtung nach einem der vorhergehenden Ansprüche, wobei der mindestens eine Speicher und der Computerprogrammcode dazu ausgelegt sind, die Vorrichtung mit dem mindestens einen Prozessor zu Folgendem zu veranlassen:
Erfassen einer Vielzahl von Typen von gemessenen Akkumulationsdaten für jedes Unterintervall, wobei die Vielzahl von Typen von Messdaten mit verschiedenen Typen von Sensoren gemessen werden;
Kombinieren der Vielzahl von Typen von gemessenen Akkumulationsdaten für jedes Unterintervall zu einem gemeinsamen Format; und
Überwachen der physischen Aktivität des Benutzers während jedes Unterintervalls durch Vergleichen der kombinierten gemessenen Akkumulationsdaten mit der entsprechenden Zielakkumulation der physischen Aktivität und durch Ausgeben eines Fortschrittsindikators, der die gemessene Akkumulation der physischen Aktivität mit Bezug auf die physische Aktivität der Zielakkumulation anzeigt.

10. Vorrichtung nach einem der vorhergehenden Ansprüche, wobei die Vorrichtung einen Bewegungssensor umfasst, der angeordnet ist, die physische Aktivität zu messen.

11. Vorrichtung nach Anspruch 10, wobei der mindestens eine Speicher und der Computerprogrammcode dazu ausgelegt sind, die Vorrichtung mit dem mindestens einen Prozessor zu veranlassen, mindestens die gemessene Akkumulation der physischen Aktivität zu einer anderen Vorrichtung zu kommunizieren.

12. Vorrichtung nach einem der vorhergehenden Ansprüche 1 bis 9, wobei der mindestens eine Speicher und der Computerprogrammcode dazu ausgelegt sind, die Vorrichtung mit dem mindestens einen Prozessor zu veranlassen, während eines überwachten Unterintervalls Aktivitätsmessdaten von einer anderen Vorrichtung zu empfangen.

13. Vorrichtung nach einem der vorhergehenden Ansprüche 1 bis 9, wobei der mindestens eine Speicher und der Computerprogrammcode dazu ausgelegt sind, die Vorrichtung mit dem mindestens einen Prozessor zu veranlassen, nach dem überwachten Unterintervall Aktivitätsmessdaten von einer anderen Vorrichtung zu empfangen und das Überwachen nach dem Empfangen der Aktivitätsmessdaten durchzuführen.

14. Computerprogrammprodukt, das sich auf einem computerlesbaren Verteilungsmedium befindet und angeordnet ist, wenn es von einem Computer gelesen wird, die Ausführung eines Computerprozesses zu veranlassen, der Folgendes umfasst:
Erfassen (200) von Zielakkumulationsparametern, die eine Zielgesamtmenge einer physischen Aktivität für einen Benutzer definiert, die innerhalb eines Beobachtungsintervalls zu akkumulieren ist, wobei die Menge der physischen Aktivität mit Bezug auf ein Attribut, das während der physischen Aktivität messbar ist, definiert ist;
Durchführen einer Zeitverteilung (202) der Zielakkumulationsparameter in eine Vielzahl von Untersätzen in einer Zeitdomäne, derart, dass jeder Untersatz mit einem Zeitunterintervall und einem Zielakkumulationswert verknüpft ist, wobei die Summe der Unterintervalle gleich dem Beobachtungsintervall ist; und
Überwachen (204) der physischen Aktivität des Benutzers während eines Unterintervalls durch Vergleichen einer gemessenen Akkumulation der physischen Aktivität mit der Zielakkumulation, die mindestens aus dem Zielakkumulationswert des Unterintervalls abgeleitet wird, und durch Ausgeben eines Fortschrittsindikators, der die gemessene Akkumulation der physischen Aktivität mit Bezug auf die Zielakkumulation anzeigt
**dadurch gekennzeichnet, dass** der Computerprozess ferner Folgendes umfasst:
Bestimmen (700) einer Zeitverteilung der gemessenen Akkumulation der physischen Aktivität über mindestens ein vorheriges Beobachtungsintervall, das dem Beobachtungsintervall vorausgeht, durch Bestimmen der gemessenen Akkumulation der physischen Aktivität während jedes Unterintervalls des mindestens einen vorherigen Beobachtungsintervalls; und
Modifizieren (702, 704) der Zeitverteilung der Zielakkumulationsparameter in der Vielzahl von Untersätzen zur bestimmten Verteilung der gemessenen Akkumulation der physischen Aktivität während des mindestens einen vorherigen Beobachtungsintervalls.

15. Vorrichtung (1100, 1102), die Folgendes umfasst:
mindestens einen Prozessor (100); und
mindestens einen Speicher (120), der Computerprogrammcode (128) beinhaltet, wobei der mindestens eine Speicher und der Computerprogrammcode dazu ausgelegt sind, die Vorrichtung mit dem mindestens einen Prozessor zu Folgendem zu veranlassen:
Erfassen (200) von Zielakkumulationsparametern, die eine Zielgesamtmenge einer physischen Aktivität für einen Benutzer definiert, die innerhalb eines Beobachtungsintervalls zu akkumulieren ist, wobei die Menge der physischen Aktivität mit Bezug auf ein Attribut, das während der physischen Aktivität messbar ist, definiert ist;
Durchführen einer Zeitverteilung (202) der Zielakkumulationsparameter in eine Vielzahl von Untersätzen in einer Zeitdomäne, derart, dass jeder Untersatz mit einem Zeitunterintervall und einem Zielakkumulationswert verknüpft ist, wobei die Summe der Unterintervalle gleich dem Beobachtungsintervall ist; und
Überwachen (204) der physischen Aktivität des Benutzers während eines Unterintervalls durch Vergleichen einer gemessenen Akkumulation der physischen Aktivität mit der Zielakkumulation, die mindestens aus dem Zielakkumulationswert des Unterintervalls abgeleitet wird, und durch Ausgeben eines Fortschrittsindikators, der die gemessene Akkumulation der physischen Aktivität mit Bezug auf die Zielakkumulation anzeigt,
**dadurch gekennzeichnet, dass** der mindestens eine Speicher und der Computerprogrammcode dazu ausgelegt sind, die Vorrichtung mit dem mindestens einen Prozessor ferner zu Folgendem zu veranlassen:
Bestimmen (500) einer Zeitverteilung der gemessenen Akkumulation der physischen Aktivität über mindestens ein vorheriges Beobachtungsintervall, das dem Beobachtungsintervall vorausgeht, durch Bestimmen der gemessenen Akkumulation der physischen Aktivität während jedes Unterintervalls des mindestens einen vorherigen Beobachtungsintervalls; und
Durchführen der Zeitverteilung (504) der Zielakkumulationsparameter in die Vielzahl von Untersätzen in der Zeitdomäne, derart, dass die Zeitverteilung (504) der Zielakkumulationsparameter der bestimmten Zeitverteilung der gemessenen Akkumulation der physischen Aktivität während des mindestens einen vorherigen Beobachtungsintervalls folgt.

16. Computerprogrammprodukt, das sich auf einem computerlesbaren Verteilungsmedium befindet und angeordnet ist, wenn es von einem Computer gelesen wird, die Ausführung eines Computerprozesses zu veranlassen, der Folgendes umfasst:
Erfassen (200) von Zielakkumulationsparametern, die eine Zielgesamtmenge einer physischen Aktivität für einen Benutzer definiert, die innerhalb eines Beobachtungsintervalls zu akkumulieren ist, wobei die Menge der physischen Aktivität mit Bezug auf ein Attribut, das während der physischen Aktivität messbar ist, definiert ist;
Durchführen einer Zeitverteilung (202) der Zielakkumulationsparameter in eine Vielzahl von Untersätzen in einer Zeitdomäne, derart, dass jeder Untersatz mit einem Zeitunterintervall und einem Zielakkumulationswert verknüpft ist, wobei die Summe der Unterintervalle gleich dem Beobachtungsintervall ist; und
Überwachen (204) der physischen Aktivität des Benutzers während eines Unterintervalls durch Vergleichen einer gemessenen Akkumulation der physischen Aktivität mit der Zielakkumulation, die mindestens aus dem Zielakkumulationswert des Unterintervalls abgeleitet wird, und durch Ausgeben eines Fortschrittsindikators, der die gemessene Akkumulation der physischen Aktivität mit Bezug auf die Zielakkumulation anzeigt
**dadurch gekennzeichnet, dass** der Computerprozess ferner Folgendes umfasst:
Bestimmen (500) einer Zeitverteilung der gemessenen Akkumulation der physischen Aktivität über mindestens ein vorheriges Beobachtungsintervall, das dem Beobachtungsintervall vorausgeht, durch Bestimmen der gemessenen Akkumulation der physischen Aktivität während jedes Unterintervalls des mindestens einen vorherigen Beobachtungsintervalls; und
Durchführen der Zeitverteilung (504) der Zielakkumulationsparameter in die Vielzahl von Untersätzen in der Zeitdomäne, derart, dass die Zeitverteilung (504) der Zielakkumulationsparameter der bestimmten Zeitverteilung der gemessenen Akkumulation der physischen Aktivität während des mindestens einen vorherigen Beobachtungsintervalls folgt.

## Revendications

1. Appareil (1100, 1102) comprenant :
au moins un processeur (100) ; et
au moins une mémoire (120) incluant un code de programme d'ordinateur (128), dans lequel l'au moins une mémoire et le code de programme d'ordinateur sont configurés, avec l'au moins un processeur, pour amener l'appareil à :
acquérir (200) des paramètres d'accumulation cible définissant une quantité totale cible d'activité physique pour un utilisateur à accumuler au sein d'un intervalle d'observation, dans lequel la quantité d'activité physique est définie en termes d'attribut mesurable pendant l'activité physique ;
réaliser une distribution temporelle (202) des paramètres d'accumulation cible dans une pluralité de sous-ensembles dans un domaine temporel de telle sorte que chaque sous-ensemble est associé à un sous-intervalle temporel et à une valeur d'accumulation cible, dans lequel la somme des sous-intervalles est égale à l'intervalle d'observation ; et
surveiller (204) l'activité physique de l'utilisateur pendant un sous-intervalle en comparant une accumulation mesurée de l'activité physique avec l'accumulation cible dérivée d'au moins la valeur d'accumulation cible du sous-intervalle et en délivrant un indicateur de progression indiquant l'accumulation mesurée de l'activité physique par rapport à l'accumulation cible,
**caractérisé en ce que** l'au moins une mémoire et le code de programme d'ordinateur sont configurés, avec l'au moins un processeur, pour amener l'appareil à en outre :
déterminer (700) une distribution temporelle de l'accumulation mesurée de l'activité physique sur au moins un intervalle d'observation antérieur précédant l'intervalle d'observation en déterminant l'accumulation mesurée de l'activité physique pendant chaque sous-intervalle de l'au moins un intervalle d'observation antérieur ; et
modifier (702, 704) la distribution temporelle des paramètres d'accumulation cible dans la pluralité de sous-ensembles vers la distribution temporelle déterminée de l'accumulation mesurée de l'activité physique pendant l'au moins un intervalle d'observation antérieur.

2. Appareil selon la revendication 1, dans lequel les valeurs d'accumulation cible représentent une fonction de distribution cumulative de la quantité totale d'activité physique, et l'au moins une mémoire et le code de programme d'ordinateur sont configurés, avec l'au moins un processeur, pour amener l'appareil à :
acquérir une accumulation mesurée de l'activité physique mesurée pendant ledit sous-intervalle et pendant tous les sous-intervalles antérieurs de l'intervalle d'observation, le cas échéant ; et
calculer l'indicateur de progression en comparant la différence entre l'accumulation mesurée acquise de l'activité physique et la valeur d'accumulation cible du sous-intervalle actuel.

3. Appareil selon la revendication 1 ou 2, dans lequel l'au moins une mémoire et le code de programme d'ordinateur sont configurés, avec l'au moins un processeur, pour amener l'appareil à réaliser la distribution temporelle (202) des paramètres d'accumulation cible dans la pluralité de sous-ensembles de manière inégale de telle sorte qu'au moins un des sous-intervalles est associé à une accumulation cible d'activité physique plus élevée qu'au moins un autre sous-intervalle.

4. Appareil selon la revendication 3, dans lequel l'au moins une mémoire et le code de programme d'ordinateur sont configurés, avec l'au moins un processeur, pour amener l'appareil à réaliser la distribution temporelle (202) des paramètres d'accumulation cible dans la pluralité de sous-ensembles de manière inégale selon une estimation initiale d'une distribution d'accumulation d'activité d'utilisateur pendant l'intervalle d'observation.

5. Appareil selon la revendication 1, dans lequel l'au moins une mémoire et le code de programme d'ordinateur sont configurés, avec l'au moins un processeur, pour amener l'appareil à décaler la distribution temporelle des paramètres d'accumulation cible de l'intervalle d'observation de telle sorte que la distribution des paramètres d'accumulation cible de l'intervalle d'observation est avancée par rapport à l'accumulation mesurée au sein de l'au moins un intervalle d'observation antérieur à raison d'un sous-intervalle.

6. Appareil selon la revendication 1 ou 5, dans lequel l'au moins une mémoire et le code de programme d'ordinateur sont configurés, avec l'au moins un processeur, pour amener l'appareil à établir une moyenne de l'accumulation mesurée de l'activité physique sur une pluralité desdits intervalles d'observation antérieurs.

7. Appareil selon une quelconque revendication précédente, dans lequel l'au moins une mémoire et le code de programme d'ordinateur sont configurés, avec l'au moins un processeur, pour amener l'appareil à :
acquérir un modèle de distribution initiale pour les paramètres d'accumulation cible ;
acquérir une valeur d'accumulation mesurée de l'activité physique pour chaque sous-intervalle mesuré pendant l'intervalle d'observation antérieur ;
déterminer un sous-intervalle dans lequel une valeur d'accumulation cible du modèle de distribution initiale est le plus élevé par rapport à la valeur d'accumulation mesurée d'un sous-intervalle ultérieur et réduisant la valeur d'accumulation cible du sous-intervalle déterminé dans le modèle de distribution initiale ;
déterminer un sous-intervalle dans lequel une valeur d'accumulation cible du modèle de distribution initiale est le plus bas par rapport à la valeur d'accumulation mesurée d'un sous-intervalle ultérieur et relève la valeur d'accumulation cible du sous-intervalle déterminé dans le modèle de distribution initiale.

8. Appareil selon une quelconque revendication précédente, dans lequel l'au moins une mémoire et le code de programme d'ordinateur sont configurés, avec l'au moins un processeur, pour amener l'appareil à acquérir l'activité physique accumulée mesurée qui comprend au moins un des attributs suivants de l'utilisateur : fréquence cardiaque, accélération, vitesse, puissance et consommation d'énergie.

9. Appareil selon une quelconque revendication précédente, dans lequel l'au moins une mémoire et le code de programme d'ordinateur sont configurés, avec l'au moins un processeur, pour amener l'appareil à :
acquérir une pluralité de types de données d'accumulation mesurée pour chaque sous-intervalle, dans lequel la pluralité de types de données de mesure sont mesurés avec différents types de capteurs ;
combiner la pluralité de types de données d'accumulation mesurée dans un format commun pour chaque sous-intervalle ; et
surveiller l'activité physique de l'utilisateur pendant chaque sous-intervalle en comparant les données d'accumulation mesurée combinées avec l'accumulation cible correspondante de l'activité physique et en délivrant un indicateur de progression indiquant l'accumulation mesurée de l'activité physique par rapport à l'activité physique d'accumulation cible.

10. Appareil selon une quelconque revendication précédente, dans lequel l'appareil comprend un capteur de mouvements agencé pour mesurer l'activité physique.

11. Appareil selon la revendication 10, dans lequel l'au moins une mémoire et le code de programme d'ordinateur sont configurés, avec l'au moins un processeur, pour amener l'appareil à communiquer au moins l'accumulation mesurée de l'activité physique à un autre appareil.

12. Appareil selon une quelconque revendication précédente 1 à 9, dans lequel l'au moins une mémoire et le code de programme d'ordinateur sont configurés, avec l'au moins un processeur, pour amener l'appareil à recevoir des données de mesure d'activité en provenance d'un autre appareil pendant le sous-intervalle surveillé.

13. Appareil selon une quelconque revendication précédente 1 à 9, dans lequel l'au moins une mémoire et le code de programme d'ordinateur sont configurés, avec l'au moins un processeur, pour amener l'appareil à recevoir des données de mesure d'activité en provenance d'un autre appareil après le sous-intervalle surveillé et réalisant ladite surveillance lors de la réception des données de mesure d'activité.

14. Produit de programme d'ordinateur mis en œuvre sur un support de distribution lisible par ordinateur agencé, lorsqu'il est lu par un ordinateur, pour amener l'exécution d'un processus d'ordinateur comprenant :
l'acquisition (200) de paramètres d'accumulation cible définissant une quantité totale cible d'activité physique pour un utilisateur à accumuler au sein d'un intervalle d'observation, dans lequel la quantité d'activité physique est définie en termes d'attribut mesurable pendant l'activité physique ;
la réalisation d'une distribution temporelle (202) des paramètres d'accumulation cible dans une pluralité de sous-ensembles dans un domaine temporel de telle sorte que chaque sous-ensemble est associé à un sous-intervalle temporel et à une valeur d'accumulation cible, dans lequel la somme des sous-intervalles est égale à l'intervalle d'observation ; et
la surveillance (204) de l'activité physique de l'utilisateur pendant un sous-intervalle en comparant une accumulation mesurée de l'activité physique avec l'accumulation cible dérivée d'au moins la valeur d'accumulation cible du sous-intervalle et en délivrant un indicateur de progression indiquant l'accumulation mesurée de l'activité physique par rapport à l'accumulation cible,
**caractérisé en ce que** le processus d'ordinateur comprend en outre :
la détermination (700) d'une distribution temporelle de l'accumulation mesurée de l'activité physique sur au moins un intervalle d'observation antérieur précédant l'intervalle d'observation en déterminant l'accumulation mesurée de l'activité physique pendant chaque sous-intervalle de l'au moins un intervalle d'observation antérieur ; et
la modification (702, 704) de la distribution temporelle des paramètres d'accumulation cible dans la pluralité de sous-ensembles vers la distribution déterminée de l'accumulation mesurée de l'activité physique pendant l'au moins un intervalle d'observation antérieur.

15. Appareil (1100, 1102) comprenant :
au moins un processeur (100) ; et
au moins une mémoire (120) incluant un code de programme d'ordinateur (128), dans lequel l'au moins une mémoire et le code de programme d'ordinateur sont configurés, avec l'au moins un processeur, pour amener l'appareil à :
acquérir (200) des paramètres d'accumulation cible définissant une quantité totale cible d'activité physique pour un utilisateur à accumuler au sein d'un intervalle d'observation, dans lequel la quantité d'activité physique est définie en termes d'attribut mesurable pendant l'activité physique ;
réaliser une distribution temporelle (202) des paramètres d'accumulation cible dans une pluralité de sous-ensembles dans un domaine temporel de telle sorte que chaque sous-domaine est associé à un sous-intervalle temporel et à une valeur d'accumulation cible, dans lequel la somme des sous-intervalles est égale à l'intervalle d'observation ; et
surveiller (204) l'activité physique de l'utilisateur pendant un sous-intervalle en comparant une accumulation mesurée de l'activité physique avec l'accumulation cible dérivée d'au moins la valeur d'accumulation cible du sous-intervalle et en délivrant un indicateur de progression indiquant l'accumulation mesurée de l'activité physique par rapport à l'accumulation cible,
**caractérisé en ce que** l'au moins une mémoire et le code de programme d'ordinateur sont configurés, avec l'au moins un processeur, pour amener l'appareil à en outre :
déterminer (500) une distribution temporelle de l'accumulation mesurée de l'activité physique sur au moins un intervalle d'observation antérieur précédant l'intervalle d'observation en déterminant l'accumulation mesurée de l'activité physique pendant chaque sous-intervalle de l'au moins un intervalle d'observation antérieur ; et
réaliser la distribution temporelle (504) des paramètres d'accumulation cible dans la pluralité de sous-ensembles dans le domaine temporel de telle sorte que la distribution temporelle (504) des paramètres d'accumulation cible suit la distribution temporelle déterminée de l'accumulation mesurée de l'activité physique pendant l'au moins un intervalle d'observation antérieur.

16. Produit de programme d'ordinateur mis en œuvre sur un support de distribution lisible par ordinateur agencé, lorsqu'il est lu par un ordinateur, pour amener l'exécution d'un processus d'ordinateur comprenant :
l'acquisition (200) de paramètres d'accumulation cible définissant une quantité totale cible d'activité physique pour un utilisateur à accumuler au sein d'un intervalle d'observation, dans lequel la quantité d'activité physique est définie en termes d'attribut mesurable pendant l'activité physique ;
la réalisation d'une distribution temporelle (202) des paramètres d'accumulation cible dans une pluralité de sous-ensembles dans un domaine temporel de telle sorte que chaque sous-ensemble est associé à un sous-intervalle temporel et à une valeur d'accumulation cible, dans lequel la somme des sous-intervalles est égale à l'intervalle d'observation ; et
la surveillance (204) de l'activité physique de l'utilisateur pendant un sous-intervalle en comparant une accumulation mesurée de l'activité physique avec l'accumulation cible dérivée d'au moins la valeur d'accumulation cible du sous-intervalle et en délivrant un indicateur de progression indiquant l'accumulation mesurée de l'activité physique par rapport à l'accumulation cible,
**caractérisé en ce que** le processus d'ordinateur comprend en outre :
la détermination (500) d'une distribution temporelle de l'accumulation mesurée de l'activité physique sur au moins un intervalle d'observation antérieur précédant l'intervalle d'observation en déterminant l'accumulation mesurée de l'activité physique pendant chaque sous-intervalle de l'au moins un intervalle d'observation antérieur ; et
la réalisation de la distribution temporelle (504) des paramètres d'accumulation cible dans la pluralité de sous-ensembles dans le domaine temporel de telle sorte que la distribution temporelle (504) des paramètres d'accumulation cible suit la distribution temporelle déterminée de l'accumulation mesurée de l'activité physique pendant l'au moins un intervalle d'observation antérieur.
